# EUROPEAN PATENT SPECIFICATION

(11) **EP 3 811 842 B1**
(45) Date of publication and mention of the grant of the patent: **21.01.2026**
(21) Application number: 19000485.3
(22) Date of filing: 23.10.2019
(51) Int. Cl.: A61B 1/00, A61B 1/005, A61B 1/05, A61B 1/06, A61B 1/018

(54) **ENDOSCOPE**
ENDOSKOP
ENDOSCOPE

(43) Date of publication of application: 28.04.2021
(73) Proprietor: Medizinische Universität Wien, 1090 Wien (AT)
(72) Inventor: KEFURT, Ronald, 1030 Wien (AT)
(74) Representative: SONN Patentanwälte GmbH & Co KG

(56) References cited:
- WO-A1-2006/126268
- JP-A- 2000 180 735
- US-A1- 2015 031 948
- US-A1- 2017 135 557
- US-B2- 8 366 607

## Description

The invention relates to an endoscope having a distal end, a proximal end and a longitudinal axis extending from the proximal end to the distal end, the endoscope comprising:
- a flexible elongate tube,
- an illumination source positioned at the distal end for illuminating a lumen of a patient,
- an image sensor for capturing images within the illuminated lumen of the patient,
- a working channel extending through the elongate tube and terminating at the distal end,
- an articulation assembly including at least one steering cable operably coupled to the distal end such that movement of the at least one steering cable induces the flexible elongate tube to articulate relative to the longitudinal axis.

It has become well established that regular endoscopic examinations are useful for the early detection and treatment of disease of the alimentary and excretory canals and of airways, e.g., the colon, esophagus, stomach, urethra, bladder and other organ systems. A conventional imaging endoscope used for such procedures comprises a flexible, elongate tube with a fiber optic light guide that directs illuminating light from an external light source to the distal end where it illuminates the lumen to be examined. An objective lens and fiber optic imaging light guide communicating with a camera at the proximal end of the endoscope, or an imaging camera chip at the distal end, produce an image that is displayed to the operator.

Frequently, the imaging assembly at the distal end of the endoscope includes an inexpensive mass-produceable assembly of components that house one or more light emitting diodes (LEDs), an image sensor such as a CMOS solid state image sensor and low cost (e.g., plastic) lens assembly. The LEDs may be thermally coupled to a heat exchanger, and air or liquid cooled in order to remove any excess heat generated by the LEDs.

In addition, most endoscopes include one or more working channels through which medical devices such as biopsy forceps, snares, fulguration probes, and other tools may be passed.

Navigation of the endoscope through complex and serpentine paths is critical for the examination to be successful with minimum pain, side effects and risk to the patient. To this end, modern endoscopes include means for deflecting the distal end or tip of the endoscope to follow the pathway of the structure under examination and to survey targeted examination sites. Steering cables similar to bicycle brake cables (Bowden cables) are arranged within the endoscope in order to connect the distal portion of the elongate tube to a set of control means, such as control knobs or rotating wheels, at the proximal endoscope handle. By manipulating the control means, the operator is able to steer the endoscope during insertion and direct it to a region of interest.

In a variant, the cables communicate with actuators within the control cabinet. In the latter, a directional controller generates electrical control signals which are sent via a processor within the control cabinet, which generates control signals to drive the actuators in order to orient the distal end of the endoscope in the direction desired by the operator. In another variant, the distal end of the endoscope may be automatically steered, based on analysis of images from the image sensor. A joystick or other directional controller may include tactile, haptic or other sensory feedback to reflect the force against a tissue wall or to alert the operator that the endoscope may be looped. The distal tip housing provides a high degree of integration of parts, for example, clear windows for the LEDs are insert-molded into the distal tip housing to eliminate any secondary window sealing operations and to ensure a hermetic seal.

An endoscope according to the preamble of independent claim 1 is known from prior art document US 2015/031948.

A shortcoming of some endoscopes of the prior art is to be seen in that although they provide for articulating or tilting of the elongate tube in the distal portion or over the entire length thereof, rotating only the head to position the camera or optical system and other medical devices in a position favorable for examination and manipulation or surgery is not always possible.

The invention, which is defined in independent claim 1 and the dependent claims 2 - 10, thus aims to provide such functionality while at the same time not compromising the functioning of the articulating assembly.

To solve this object, an endoscope of the initially mentioned kind according to the invention has a head arranged at the distal end, with said head carrying at least the illumination source and the image sensor and comprising a distal section of the working channel, said head being rotatable relative to the flexible elongate tube about the longitudinal axis. By providing a head that offers rotatability independently from the articulating functionality of the elongate tube, these two functionalities can be mechanically uncoupled from each other so that both types of movement of the inventive endoscope can be effected without interacting with each other.

In order to allow for tilting the very distal portion of the inventive endoscope with the head, the at least one steering cable of the articulation assembly can be connected to the rotatable head itself, the head thus forming the distal end of the inventive endoscope. In this case however, the effective length of the at least one steering cable is altered by spiraling of the at least one steering cable as the head is rotated, which, in turn, undesirably alters the degree of articulation of the elongate tube. To compensate for this, the inventive endoscope according to the invention is devised in such a manner, that a compensating unit is provided for adjusting the effective length of the at least one steering cable as a function of a rotation angle of the head. The compensating unit is devised in such a manner, that additional length of steering cable can be provided by unrolling cable from a cable storage means when the head is rotated.

In order to allow for precise compensation of the effective length of the at least one steering cable, the inventive endoscope is further characterized in that a sensor is provided for sensing a rotation angle of the head relative to the flexible elongate tube, wherein the sensor is connected to the compensating unit for providing a signal that is representative of a rotation angle to the compensating unit. Depending on the signal, the compensating unit can be controlled to unroll an appropriate length of additional steering cable to compensate for the reduction in effective length caused by the rotation of the head and the thus resulting spiraling of the at least one steering cable. To this end, and in order to allow for automation of the compensation, the inventive endoscope is preferably further devised such, that the compensating unit comprises a control unit and a motor for driving an adjustment mechanism for adjusting the effective length of the at least one steering cable, wherein the control unit is configured to control the motor as a function of the signal received from the sensor.

Preferably, a rotation drive is provided for rotating the head relative to the flexible elongate tube. The rotation drive can preferably be devised as a motorized drive operably connected with a gear. Alternatively, the rotation drive may be a manual drive arrangement, such as a rotation wheel that can be rotated manually by the operator.

The articulating means may be devised in various ways. It is, however preferred, that the articulation assembly comprises at least one rotating wheel that operably couples to the at least one steering cable such that rotation of the at least one rotating wheel moves the at least one steering cable to articulate the flexible elongate tube relative to the longitudinal axis thereof. Moving the steering cable by rotating the rotating wheel preferably is effected by the rotating wheel being operably coupled to a winding shaft on which the at least on steering cable is wound up or unrolled depending on the direction of rotation of the rotating wheel.

According to a preferred embodiment of the present invention the articulation assembly comprises first and second pairs of steering cables adapted to effect articulation of the elongate tube in first and second planes respectively parallel to the longitudinal axis of the elongate tube, the first and second planes being oriented perpendicular to each other. Providing a pair of steering cables for articulation of the elongate tube in each plane, in which the elongate tube is to be articulated or deflected, provides for articulation in both directions within that plane starting from an undeflected state of the elongate tube. To this end, the steering cables within each pair of steering cables are each wound up in opposite directions on separate winding shafts as it is in accordance with a preferred embodiment of the present invention. The separate winding shafts thus each carry one pair of steering cables wound up in opposite directions. The separate winding shafts are, of course, each separately coupled to separate first and second rotating wheels. In the context of the present invention, the rotating wheels may also be addressed as control wheels. Preferably, one of the winding shafts is guided through the other winding shaft, the other winding shaft being devised as a hollow winding shaft.

In order to allow for compensation of the effective length of the at least one steering cable without influencing the degree of articulation of the elongate tube, first coupling means are provided for selectively coupling and de-coupling the rotating wheel from the at least one steering cable as it is in accordance with a preferred embodiment of the present invention. This allows for the compensating unit to adjust the effective length of the at least one steering cable without changing the rotational position of the rotating wheel.

The compensating unit is preferably comprised of third coupling and decoupling means adapted to couple and decouple the at least one winding shaft into subsections each provided with a pinion, the second coupling and decoupling means adapted to couple and decouple a spur gear adapted to be brought in and out of engagement with the pinions on the subsections of the at least on winding shaft and a motor for driving the spur gear. Rotating the spur gear in engagement with the pinions thus brings about equal winding of both steering cables of a pair of steering cables thus lengthening or shortening the cables depending on the sense of rotation of the spur wheel without changing the difference in length between the two cables that was effected when articulating the elongate tube. In the case of two pairs of steering cables wound up on separate winding shafts, two compensating units are provided for operating the first and second pairs of steering cables.

The present invention will now be exemplified in more detail by way of an exemplary embodiment represented in the drawing, in which
Fig. 1 shows a schematic sectional view of the rotating head of the inventive endoscope
Fig. 2 shows the rotating head in a schematic perspective view, and
Fig. 3 shows a schematic representation of the articulation assembly.

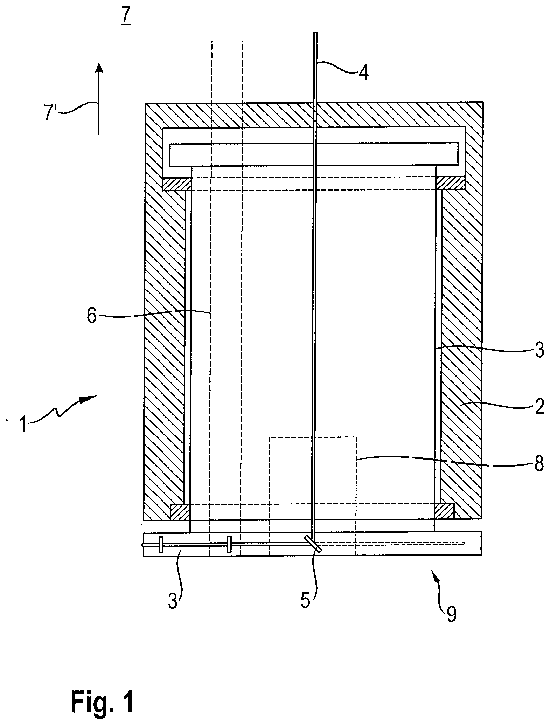

In Fig. 1, the rotating head is denoted by reference numeral 1 and it can be seen, that the rotating head 1 is generally comprised of an outer body 2 holding and rotatably guiding an inner body 3 therein. A rotation cable for rotating the inner body 3 with respect to the outer body 2 is denoted by reference numeral 4. The rotation cable 4 runs from the proximal end 7 of the endoscope 1, which proximal end 7 (not shown) is pointed at by arrow 7', to the rotating head 1 and the rotation cable 4 is deflected by a deflection ring 5 and attached to the inner body 3 to allow for rotation of the rotating head 1 or more precisely the inner body 3 thereof upon moving or pulling the rotation cable 4. A working channel 6 coming from the proximal end 7 is extending through the head 1. A camera 8 is arranged at the distal end 9 of the head 1, i.e. the distal end 9 of the endoscope.

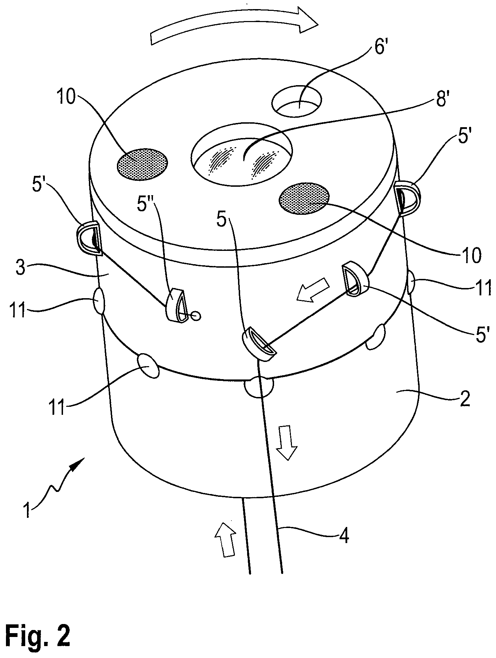

In Fig. 2, the rotating head 1 is shown to have further deflection rings 5 or guiding rings 5' for the rotation cable 4, which is attached at an attachment ring 5". An aperture 8' of the camera or image sensor 8 is surrounded by a plurality of light or illumination sources 10. Reference numeral 6' denotes an orifice of the working channel 6. The outer body 2 and the inner body 3 slide on each other by the means of a ball bearing 11. In Fig. 2, the dimensions of the inner body 3 with respect to the outer body 2 are different from those in Fig. 1 without changing the principle of operation of the head 1.

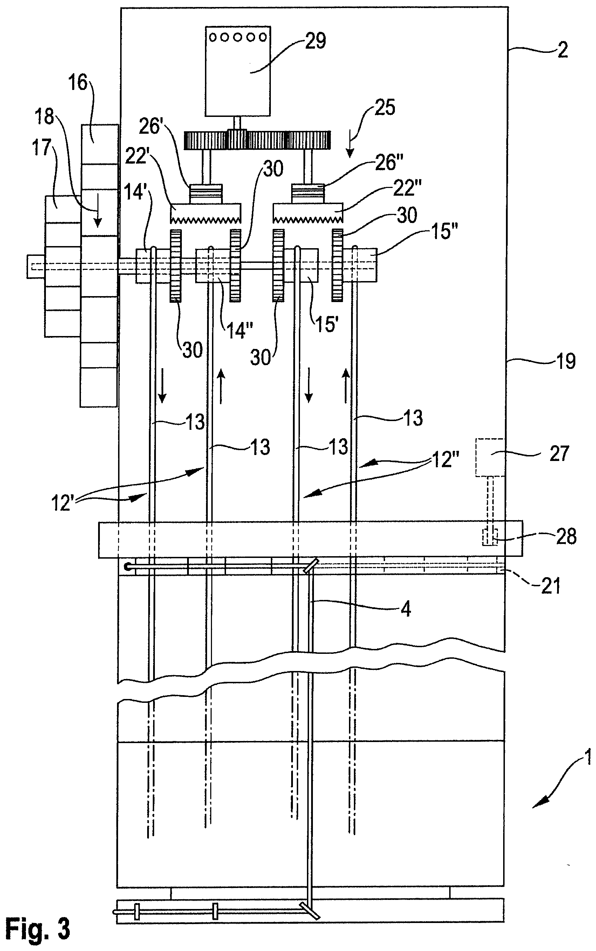

The articulation assembly and the compensating unit are depicted in Fig. 3 and it can be seen, that a first pair 12' of steering cables 13 and a second pair 12" of steering cables 13 are wound up in opposite directions on winding shafts 14', 14'' and 15', 15'' as represented by the steering cables 13 being drawn in continuous lines and dashed lines respectively on the winding shafts 14, 14', 15', 15''. The winding shafts can each be operably connected to rotating wheels 16 and 17 adapted to rotate winding shaft 14', 14'' and 15', 15'' respectively. Rotating the rotating wheel 16, for example in the sense of arrow 18, effects unrolling cable from winding shaft 14' and rolling up cable onto winding shaft 14'' thereby causing a difference in length between the steering cables 13 within the first pair 12', which causes the elongate tube 19 to articulate in a first plane. Equally, rotating the rotating wheel 17, for example also in the sense of arrow 18, effects unwinding cable from winding shaft 15' and rolling up cable from winding shaft 15'' thereby causing a difference in length between the steering cables 13 within the second pair 12", which causes the elongate tube 19 to articulate in a second plane perpendicular to the first plane. On actuation of the rotation cable 4 by means of a drive 21 for rotating the rotating head 1, the winding shafts 14', 14'' and 15', 15'' are decoupled from the rotating wheels 16 and 17 by first coupling means and decoupled into subsections to allow inverse rotation of the subsections of the winding shafts 14', 14'' and 15', 15'' while at the same time spur gears 22' and 22'' are engaging in the pinions 30 by moving the spur gears 22', 22'' in the direction of arrow 25 by second coupling and decoupling means 26', 26''. Upon rotation of the spur gears by the aid of a motor 29 the pinions 30 will be rotated in opposite directions bringing about equal winding of both steering cables 13 of a pair 12' or 12'' of steering cables 13 thus equally lengthening or shortening the steering cables 13 depending on the sense of rotation of the spur wheels 26', 26'' without changing the difference in length between the two cables 13 that was effected when articulating the elongate tube 19. Rotating the spur gears 22', 22'' is controlled by a control unit 27 in operating connection with a sensor 28 for sensing a rotation angle of the head 1 relative to the flexible elongate tube 19.

## Claims

1. An endoscope having a distal end (9), a proximal end (7) and a longitudinal axis extending from the proximal end (7) to the distal end (9), the endoscope comprising:
- a flexible elongate tube (19),
- an illumination source (10) positioned at the distal end (9) for illuminating a lumen of a patient,
- an image sensor (8) for capturing images within the illuminated lumen of the patient,
- a working channel (6) extending through the elongate tube (19) and terminating at the distal end (9),
- an articulation assembly including at least one steering cable (13) operably coupled to the distal end (9) such that movement of the at least one steering cable (13) induces the flexible elongate tube (19) to articulate relative to the longitudinal axis,
wherein a head (1) is arranged at the distal end (9), said head (1) carrying at least the illumination source (10) and the image sensor (8) and comprising a distal section of the working channel (6), said head (1) being rotatable relative to the flexible elongate tube (19) about the longitudinal axis,
**characterized in that** a compensation unit is provided for adjusting the effective length of the at least one steering cable (13) as a function of a rotation angle of the head (1), wherein a sensor (28) is provided and which is configured to sense a rotation angle of the head (1) relative to the flexible elongate tube (19), wherein the sensor (28) is connected to the compensation unit for providing a signal that is representative of a rotation angle to the compensation unit.

2. Endoscope according to claim 1, **characterized in that** a rotation drive is provided for rotating the head (1) relative to the flexible elongate tube (19).

3. Endoscope according to claim 1 or 2, **characterized in that** the compensation unit comprises a control unit (27) and a motor (29) for driving an adjustment mechanism for adjusting the effective length of the at least one steering cable (13), wherein the control unit (27) is configured to control the motor (29) as a function of the signal received from the sensor (28).

4. Endoscope according to one of claims 1 to 3, **characterized in that** the articulation assembly comprises at least one rotating wheel (16, 17) that operably couples to the at least one steering cable (13) such that rotation of the at least one rotating wheel (16, 17) moves the at least one steering cable (13) to articulate the flexible elongate tube (19) relative to the longitudinal axis thereof.

5. Endoscope according to claim 4, **characterized in that** the at least one rotating wheel (16, 17) is operably coupled to a winding shaft (14', 14'', 15', 15'') on which the at least on steering cable (13) is wound up or unrolled depending on the direction of rotation of the rotating wheel (16, 17).

6. Endoscope according to one of claims 1 to 5, **characterized in that** the articulation assembly comprises first and second pairs (12', 12'') of steering cables (13) adapted to effect articulation of the elongate tube (19) in first and second planes respectively parallel to the longitudinal axis of the elongate tube (19), the first and second planes (12', 12'') being oriented perpendicular to each other.

7. Endoscope according to claim 6, **characterized in that** the steering cables (13) within each pair (12', 12'') of steering cables (13) are each wound up in opposite directions on separate winding shafts (14', 14'', 15', 15'').

8. Endoscope according to one of claims 4 to 7, **characterized in that** first coupling means are provided for selectively coupling and de-coupling the rotating wheel (16, 17) from the at least one steering cable (13).

9. Endoscope according to one of claims 1 to 8, **characterized in that** second coupling means (26', 26'') are provided for selectively coupling and de-coupling the drive connection between the motor (29) and the at least one steering cable (13) via the adjustment mechanism.

10. Endoscope according to one of claims 3 to 9, **characterized in that** the compensating unit is comprised of third coupling and decoupling means adapted to couple and decouple the at least one winding shaft (14', 14", 15', 15") into subsections each provided with a pinion (30), the second coupling and decoupling means (26', 26") adapted to couple and decouple a spur gear (22', 22'') adapted to be brought in and out of engagement with the pinions (30) on the subsections of the at least on winding shaft (14', 14'', 15', 15'') and a motor 29 for driving the spur gear.

## Patentansprüche

1. Ein Endoskop, aufweisend ein distales Ende (9), ein proximales Ende (7) und eine Längsachse, die sich vom proximalen Ende (7) zum distalen Ende (9) erstreckt, wobei das Endoskop Folgendes umfasst:
- ein flexibles längliches Rohr (19),
- eine Beleuchtungsquelle (10), die am distalen Ende (9) positioniert ist, um ein Lumen eines Patienten zu beleuchten,
- einen Bildsensor (8) zum Erfassen von Bildern innerhalb des beleuchteten Lumens des Patienten,
- einen Arbeitskanal (6), der sich durch das längliche Rohr (19) erstreckt und am distalen Ende (9) endet,
- eine Artikulationsanordnung, die mindestens ein Steuerkabel (13) umfasst, das funktionell mit dem distalen Ende (9) gekoppelt ist, sodass eine Bewegung des mindestens einen Steuerkabels (13) bewirkt, dass sich das flexible längliche Rohr (19) relativ zur Längsachse artikuliert,
wobei ein Kopf (1) am distalen Ende (9) angeordnet ist, wobei der Kopf (1) mindestens die Beleuchtungsquelle (10) und den Bildsensor (8) trägt und einen distalen Abschnitt des Arbeitskanals (6) umfasst, wobei der Kopf (1) relativ zum flexiblen länglichen Rohr (19) um die Längsachse drehbar ist,
**dadurch gekennzeichnet, dass** eine Kompensationseinheit zum Anpassen der effektiven Länge des mindestens einen Steuerkabels (13) als Funktion eines Drehwinkels des Kopfes (1) bereitgestellt ist, wobei ein Sensor (28) bereitgestellt ist, der konfiguriert ist, einen Drehwinkel des Kopfes (1) relativ zum flexiblen länglichen Rohr (19) zu erfassen, wobei der Sensor (28) mit der Kompensationseinheit verbunden ist, um ein Signal, das repräsentativ für einen Drehwinkel ist, an die Kompensationseinheit bereitzustellen.

2. Endoskop nach Anspruch 1, **dadurch gekennzeichnet, dass** ein Drehantrieb zum Drehen des Kopfes (1) relativ zum flexiblen länglichen Rohr (19) bereitgestellt ist.

3. Endoskop nach Anspruch 1 oder 2, **dadurch gekennzeichnet, dass** die Kompensationseinheit eine Steuereinheit (27) und einen Motor (29) zum Antreiben eines Einstellmechanismus zum Einstellen der effektiven Länge des mindestens einen Steuerkabels (13) umfasst, wobei die Steuereinheit (27) dazu konfiguriert ist, den Motor (29) als Funktion des vom Sensor (28) empfangenen Signals zu steuern.

4. Endoskop nach einem der Ansprüche 1 bis 3, **dadurch gekennzeichnet, dass** die Artikulationsanordnung mindestens ein Drehrad (16, 17) umfasst, das funktionell mit dem mindestens einen Steuerkabel (13) gekoppelt ist, sodass eine Drehung des mindestens einen Drehrads (16, 17) das mindestens eine Steuerkabel (13) bewegt, um das flexible längliche Rohr (19) relativ zu dessen Längsachse zu artikulieren.

5. Endoskop nach Anspruch 4, **dadurch gekennzeichnet, dass** das mindestens eine Drehrad (16, 17) funktionell mit einer Wickelwelle (14', 14'', 15', 15'') gekoppelt ist, auf der das mindestens eine Steuerkabel (13) je nach Drehrichtung des Drehrads (16, 17) auf- oder abgewickelt wird.

6. Endoskop nach einem der Ansprüche 1 bis 5, **dadurch gekennzeichnet, dass** die Artikulationsanordnung erste und zweite Paare (12', 12'') von Steuerkabeln (13) umfasst, die geeignet sind, eine Artikulation des länglichen Rohrs (19) in ersten bzw. zweiten Ebenen parallel zur Längsachse des länglichen Rohrs (19) zu bewirken, wobei die ersten und zweiten Ebenen (12', 12'') senkrecht zueinander ausgerichtet sind.

7. Endoskop nach Anspruch 6, **dadurch gekennzeichnet, dass** die Steuerkabel (13) innerhalb jedes Paares (12', 12'') von Steuerkabeln (13) jeweils in entgegengesetzten Richtungen auf separaten Wickelwellen (14', 14'', 15', 15'') aufgewickelt sind.

8. Endoskop nach einem der Ansprüche 4 bis 7, **dadurch gekennzeichnet, dass** erste Kopplungsmittel zum selektiven Koppeln und Entkoppeln des Drehrads (16, 17) von dem mindestens einen Steuerkabel (13) bereitgestellt sind.

9. Endoskop nach einem der Ansprüche 1 bis 8, **dadurch gekennzeichnet, dass** zweite Kopplungsmittel (26', 26'') zum selektiven Koppeln und Entkoppeln der Antriebsverbindung zwischen dem Motor (29) und dem mindestens einen Steuerkabel (13) über den Einstellmechanismus bereitgestellt sind.

10. Endoskop nach einem der Ansprüche 3 bis 9, **dadurch gekennzeichnet, dass** die Kompensationseinheit dritte Kopplungs- und Entkopplungsmittel umfasst, die geeignet sind, die mindestens eine Wickelwelle (14', 14'', 15', 15'') in Unterabschnitte zu koppeln und zu entkoppeln, wobei jeder mit einem Ritzel (30) versehen ist, wobei die zweiten Kopplungs- und Entkopplungsmittel (26', 26'') geeignet sind, ein Stirnrad (22', 22'') zu koppeln und zu entkoppeln, das in und außer Eingriff mit den Ritzeln (30) an den Unterabschnitten der mindestens einen Wickelwelle (14', 14'', 15', 15'') gebracht werden kann, und einen Motor (29) zum Antreiben des Stirnrads.

## Revendications

1. Un endoscope ayant une extrémité distale (9), une extrémité proximale (7) et un axe longitudinal s'étendant de l'extrémité proximale (7) à l'extrémité distale (9), l'endoscope comprenant:
- un tube allongé flexible (19),
- une source d'éclairage (10) positionnée à l'extrémité distale (9) pour éclairer une lumière d'un patient,
- un capteur d'image (8) pour capturer des images à l'intérieur de la lumière éclairée du patient,
- un canal opérateur (6) s'étendant à travers le tube allongé (19) et se terminant à l'extrémité distale (9),
- un ensemble d'articulation comprenant au moins un câble de direction (13) couplé fonctionnellement à l'extrémité distale (9) de sorte que le mouvement dudit au moins un câble de direction (13) induit le tube allongé flexible (19) à s'articuler par rapport à l'axe longitudinal,
dans lequel une tête (1) est disposée à l'extrémité distale (9), ladite tête (1) portant au moins la source d'éclairage (10) et le capteur d'image (8) et comprenant une section distale du canal opérateur (6), ladite tête (1) étant rotative par rapport au tube allongé flexible (19) autour de l'axe longitudinal,
**caractérisé en ce qu'**une unité de compensation est prévue pour ajuster la longueur effective dudit au moins un câble de direction (13) en fonction d'un angle de rotation de la tête (1), un capteur (28) etant prévu et configuré pour détecter un angle de rotation de la tête (1) par rapport au tube allongé flexible (19), le capteur (28) etang connecté à l'unité de compensation afin de fournir un signal représentatif d'un angle de rotation à l'unité de compensation.

2. Endoscope selon la revendication 1, **caractérisé en ce qu'**un entraînement de rotation est prévu pour faire tourner la tête (1) par rapport au tube allongé flexible (19).

3. Endoscope selon la revendication 1 ou 2, **caractérisé en ce que** l'unité de compensation comprend une unité de contrôle (27) et un moteur (29) pour entraîner un mécanisme d'ajustement pour ajuster la longueur effective dudit au moins un câble de direction (13), où l'unité de contrôle (27) est configurée pour contrôler le moteur (29) en fonction du signal reçu du capteur (28).

4. Endoscope selon l'une des revendications 1 à 3, **caractérisé en ce que** l'ensemble d'articulation comprend au moins une roue rotative (16, 17) fonctionnellement couplée audit au moins un câble de direction (13) de sorte que la rotation de ladite au moins une roue rotative (16, 17) déplace ledit au moins un câble de direction (13) pour articuler le tube allongé flexible (19) par rapport à son axe longitudinal.

5. Endoscope selon la revendication 4, **caractérisé en ce que** ladite au moins une roue rotative (16, 17) est couplée fonctionnellement à un arbre d'enroulement (14', 14'', 15', 15'') sur lequel ledit au moins un câble de direction (13) est enroulé ou déroulé selon le sens de rotation de la roue rotative (16, 17).

6. Endoscope selon l'une des revendications 1 à 5, **caractérisé en ce que** l'ensemble d'articulation comprend des première et seconde paires (12', 12'') de câbles de direction (13) adaptés pour articuler le tube allongé (19) dans des premier et second plans respectivement parallèles à l'axe longitudinal du tube allongé (19), les premier et second plans (12', 12'') étant orientés perpendiculairement l'un à l'autre.

7. Endoscope selon la revendication 6, **caractérisé en ce que** les câbles de direction (13) au sein de chaque paire (12', 12'') de câbles de direction (13) sont chacun enroulés dans des directions opposées sur des arbres d'enroulement séparés (14', 14'', 15', 15'').

8. Endoscope selon l'une des revendications 4 à 7, **caractérisé en ce que** des premiers moyens de couplage sont prévus pour coupler et découpler sélectivement la roue rotative (16, 17) dudit au moins un câble de direction (13).

9. Endoscope selon l'une des revendications 1 à 8, **caractérisé en ce que** des seconds moyens de couplage (26', 26'') sont prévus pour coupler et découpler sélectivement la liaison d'entraînement entre le moteur (29) et ledit au moins un câble de direction (13) via le mécanisme d'ajustement.

10. Endoscope selon l'une des revendications 3 à 9, **caractérisé en ce que** l'unité de compensation comprend des troisièmes moyens de couplage et de découplage adaptés pour coupler et découpler ledit au moins un arbre d'enroulement (14', 14'', 15', 15'') en sous-sections chacune pourvue d'un pignon (30), les seconds moyens de couplage et de découplage (26', 26'') adaptés pour coupler et découpler un engrenage à denture droite (22', 22'') adapté pour être mis en engagement et hors engagement avec les pignons (30) sur les sous-sections dudit au moins un arbre d'enroulement (14', 14'', 15', 15'') et un moteur (29) pour entraîner l'engrenage à denture droite.
